# EUROPEAN PATENT SPECIFICATION

(11) **EP 3 646 833 B1**
(45) Date of publication and mention of the grant of the patent: **18.06.2025**
(21) Application number: 18844571.2
(22) Date of filing: 05.07.2018
(51) Int. Cl.: A61F 13/49, A61F 13/15, A61F 13/496

(54) **UNDERPANTS-SHAPED ABSORBENT ARTICLE**
ABSORBIERENDER HOCHZIEHARTIKEL
ARTICLE ABSORBANT À REMONTER

(30) Priority: 08.08.2017 JP 2017153647
(43) Date of publication of application: 06.05.2020
(73) Proprietor: Unicharm Corporation, Shikokuchuo-shi, Ehime 799-0111 (JP)
(72) Inventor: MUKAI, Hirotomo, Kanonji-shi Kagawa 769-1602 (JP)
(74) Representative: Dolleymores
(86) International application number: PCT/JP2018/025540
(87) International publication number: WO 2019/031124

(56) References cited:
- EP-A1- 2 529 715
- EP-A1- 3 117 809
- EP-A1- 3 544 563
- WO-A1-2013/094591
- WO-A1-2018/097776
- JP-A- 2001 333 932
- JP-A- 2007 075 277
- JP-A- 2007 075 277
- JP-A- 2014 233 373
- JP-A- 2016 059 608
- US-A1- 2016 166 444

## Description

### [Technical Field]

The present invention relates to an underpants-shaped absorbent article.

### [Background Art]

As an underpants-shaped absorbent article, for example, PTL 1 discloses a pull-on disposable diaper including an absorbent main body and a tubular waist portion in which two lateral side edges of a front exterior sheet that covers the front side of a wearer are joined to two lateral side edges of a back exterior sheet that covers the back side of the wearer.

In the diaper of PTL 1, the back exterior sheet includes a back extending portion extending on the lower side of the joint portion. The back extending portion includes a buttock-covering portion extending on two lateral outer sides of the absorbent main body. The buttock-covering portion is provided with a plurality of elastic members fixed extending in the lateral direction with an interval therebetween in the up-down direction. Thus, the wearer's buttocks can be covered with the buttock-covering portion. In addition, protrusion and turning-up of the buttock-covering portion can be avoided by the elastic members, and the buttock-covering portion can be caused to fit the wearer's buttocks. EP2529715A1, EP3117809A1 and US 2016/166444A1 disclose absorbent articles and/or methods relating to the same.

### [Citation List]

### [Patent Literature]

[PTL 1] Japanese Patent Application Publication No. 2008-212249

### [Summary of Invention]

### [Technical Problem]

However, the width of the buttock-covering portion extending on one side of the absorbent main body is smaller than the width of the absorbent main body, and accordingly a target wearer of the diaper of PTL 1 is a child. In an adult diaper compared with a child diaper, it is necessary to increase the width of the buttock-covering portion. Incidentally, the degree of the curvature of the buttocks of an adult is large. Therefore, compared with a child diaper, it is difficult to cause the buttock-covering portion to fit a wearer. Accordingly, when the diaper of PTL 1 is employed for adult use, it is difficult for the laterally-extending elastic members alone to fit the buttock-covering portion to a wearer.

The present invention was achieved in light of conventional problems such as that described above, and an aspect of the present invention is to provide an underpants-shaped absorbent article that fits the wearer's buttocks.

### [Solution to Problem]

A main aspect of the present invention for achieving the above-described aspect is defined in claim 1.

### [Advantageous Effects of Invention]

According to the present invention, it is possible to provide an underpants-shaped absorbent article that fits the wearer's buttocks.

### [Brief Description of the Drawings]

[FIG. 1] FIG. 1 is a schematic front view of a pull-on disposable diaper 1 (hereinafter "diaper") as viewed from the front side.
[FIG. 2] FIG. 2 is a schematic plan view of the diaper 1 in an unfolded and stretched state as viewed from a non-skin side.
[FIG. 3] FIG. 3 is a schematic cross-sectional view taken along line A-A of FIG. 2.
[FIG. 4] FIG. 4 is an illustration of an arrangement of inclining elastic members 37 and lateral elastic members 38 in an outer extending portion 322.
[FIG. 5] FIG. 5 is an illustration of an arrangement of waist elastic members 36 and the lateral elastic members 38 in a back waist member 30.
[FIG. 6] FIG. 6 is a schematic plan view of the diaper 1 of a first modified example in an unfolded and stretched state as viewed from a skin side.
[FIG. 7] FIG. 7 is a schematic plan view of the diaper 1 of a second modified example in an unfolded and stretched state as viewed from the skin side.

### [Description of Embodiments]

At least the following matters will become clear with the description of this specification and the attached drawings.

An underpants-shaped absorbent article having an up-down direction, a left-right direction, and a front-back direction,
the underpants-shaped absorbent article including:
an absorbent main body;
a front waist member; and
a back waist member,
the front waist member and the back waist member being joined to each other in two side end portions in the left-right direction by a pair of joint portions,
the back waist member including an extending portion that extends downward with respect to the joint portions,
   the extending portion including an inclining elastic member and a lateral elastic member,
      the inclining elastic member being inclined with respect to the left-right direction, in a left-right-direction side end portion of the extending portion,
      the lateral elastic member extending in the left-right direction,
   when viewed in the front-back direction, the inclining elastic member and the lateral elastic member having an overlapping portion where the inclining elastic member and the lateral elastic member overlap each other,
   concerning a length from a left-right-direction side end of the absorbent main body on one side to a left-right-direction side end of the back waist member on the one side,
      the length being longer than a left-right-direction length of the absorbent main body in a state in which the underpants-shaped absorbent article is stretched in the left-right direction.

According to such an underpants-shaped absorbent article, due to the long length of the extending portion in the left-right direction and the wide area of the extending portion, it is possible to cover the wearer's buttocks even with large buttocks. In other words, a wide portion of the wearer's buttocks can be covered by the soft extending portion having low stiffness compared with the absorbent main body, and this improves comfort. In addition, due to the inclining elastic members causing the left-right-direction side end portions of the extending portion to fit along the outline of the wearer's buttocks, namely, due to the elastic members positioned on the outer side of an apex portion of the buttocks, it is possible for the extending portion to wrap the wearer's buttocks while the extending portion is not turned up even though the area of the extending portion is large. In particular, the stiffness of the left-right-direction side end portions of the extending portion increases due to an overlapping portion of the inclining elastic members and the lateral elastic members. This makes it easier to maintain a state in which the side end portions of the extending portion fit along the outline of the wearer's buttocks. This makes it less likely for the extending portion to turn up or displace even when the wearer moves. In addition, even when the area of the extending portion is large, the extending portion extends along the wearer's buttocks in close contact therewith without floating due to the lateral elastic members.

In such an underpants-shaped absorbent article, it is desirable that
the lateral elastic member extends outward in the left-right direction with respect to the inclining elastic member.

According to such an underpants-shaped absorbent article, the stiffness of the left-right-direction side end portions of the extending portion increases due to the overlapping portion of the inclining elastic members and the lateral elastic members. This makes it easier to maintain a state in which the side end portions of the extending portion fit along the outline of the wearer's buttocks. In addition, portions outside the inclining elastic members in the left-right direction contract due to the lateral elastic members, and thereby the left-right-direction side end portions of the extending portion are formed into a frill shape. Consequently, texture of the side end edge of the extending portion is improved. Moreover, it is also possible to provide a user with a visually soft impression.

In such an underpants-shaped absorbent article, it is desirable that
the lateral elastic member reaches a left-right-direction side end of the extending portion.

According to such an underpants-shaped absorbent article, a larger stretching force of the lateral elastic members is exerted on regions outside the inclining elastic members in the left-right direction, increasing the size of the recesses and protrusions of frills. Consequently, favorable texture is available during wearing, and the visual impression of the user can be further improved by the large frills.

In such an underpants-shaped absorbent article, it is desirable
that a plurality of the lateral elastic members are disposed in the extending portion at an interval in the up-down direction,
that a lower end of the inclining elastic member extends downward beyond a lowermost one of the plurality of lateral elastic members.

According to such an underpants-shaped absorbent article, the inclining elastic members extend to the lower side. This makes it easier to suppress turning-up and displacement of the lower part of the extending portion, enabling the lower part of the extending portion to fit the wearer's buttocks.

In such an underpants-shaped absorbent article, it is desirable
that the inclining elastic member inclines downward in the up-down direction from an outer side toward an inner side in the left-right direction, and

that the inclining elastic member does not reach a side end of the absorbent main body in the left-right direction.

According to such an underpants-shaped absorbent article, a region in which no inclining elastic members are disposed is present at the lower end portion of the extending portion. This makes it difficult for the stretching force of the inclining elastic members to be exerted directly on the absorbent main body, making it possible to suppress displacement of the absorbent main body due to being stretched during wearing. Moreover, even when the wearer moves the legs, the line of the inclining elastic members disposed at the buttocks can be suppressed from being twisted and causing the extending portion to be turned up.

In such an underpants-shaped absorbent article, it is desirable
that in a packaged state of the underpants-shaped absorbent article,
   the front waist member and the back waist member are folded at a folding position extending in the up-down direction, toward an inner side in the left-right direction, and
that the inclining elastic member and the folding position do not overlap each other when viewed in the front-back direction.

According to such an underpants-shaped absorbent article, it is possible to suppress a crease extending in the up-down direction from being more likely to be formed in the inclining elastic members. In addition, appropriate stretchability can be imparted to the outer extending portion. Therefore, due to the inclining elastic members, it becomes easier for the side end portions of the extending portion to fit along the outline of the wearer's buttocks.

In such an underpants-shaped absorbent article, it is desirable
that the back waist member includes a waist portion on an upper side of the extending portion,
that the waist portion includes a waist elastic member extending in the left-right direction, and
that the waist elastic member is disposed inside the joint portion in the left-right direction.

According to such an underpants-shaped absorbent article, it is possible to suppress the stiffness of the joint portion from being further increased as a result that the waist elastic member overlaps the joint portion whose stiffness has been increased by means such as welding. Thus, degradation in sense of comfort in the diaper 1 can be avoided.

In such an underpants-shaped absorbent article, it is desirable
that a plurality of the waist elastic members are disposed in the waist portion at an interval in the up-down direction,
that a plurality of the lateral elastic members are disposed in the extending portion at an interval in the up-down direction, and
that an interval in the up-down direction between the waist elastic members adjacent in the up-down direction is equal to an interval in the up-down direction between the lateral elastic members adjacent in the up-down direction.

According to such an underpants-shaped absorbent article, it is possible to dispose the elastic members of two different types at an equal pitch, making it possible to simplify adjusting the positions of the elastic members in a manufacturing process. This facilitates manufacturing of the back waist member 30, and allows to suppress increase of manufacturing costs.

In such an underpants-shaped absorbent article, it is desirable
that a plurality of the waist elastic members are disposed in the waist portion at an interval in the up-down direction,
that a plurality of the lateral elastic members are disposed in the extending portion at an interval in the up-down direction, and
that an interval in the up-down direction between the lateral elastic members adjacent in the up-down direction is larger than an interval in the up-down direction between the waist elastic members adjacent in the up-down direction.

According to such an underpants-shaped absorbent article, the pitch of the lateral elastic members in the up-down direction increases, and therefore the stretching force which the lateral elastic members exerts on a unit area of the extending portion decreases. Consequently, a fastening force exerted by the extending portion decreases when the absorbent article is put on, and this prevents the force for fastening at the buttocks from being excessively strong, making it possible to give appropriate fitting to the wearer.

In such an underpants-shaped absorbent article, it is desirable
that a plurality of the waist elastic members are disposed in the waist portion at an interval in the up-down direction,
that a plurality of the lateral elastic members are disposed in the extending portion at an interval in the up-down direction, and
that an interval in the up-down direction between the lateral elastic members adjacent in the up-down direction is smaller than an interval in the up-down direction between the waist elastic members adjacent in the up-down direction.

According to such an underpants-shaped absorbent article, the pitch of the lateral elastic members in the up-down direction decreases, and therefore a stretching force by the lateral elastic members easily exerts on the entirety of the extending portion. This makes it easier for the extending portion to fit the wearer's buttocks in surface-to-surface contact. Consequently, it is more likely to suppress separating away from the extending portion during wearing.

In such an underpants-shaped absorbent article, it is desirable
that a plurality of the inclining elastic members are disposed in the extending portion at a gap in the left-right direction,
that a maximum value of a left-right-direction gap between a left-right-direction outermost pair of the inclining elastic members adjacent to each other in the left-right direction
is smaller than
a minimum value of a left-right-direction gap between a pair of the inclining elastic members adjacent to each other in the left-right direction located inside the outermost pair in the left-right direction.

According to such an underpants-shaped absorbent article, the gap between the inclining elastic members disposed close to the left-right-direction outermost outer edge portions of the extending portion is narrow, and thus, the stretching force of the inclining elastic members concentrates and is easily exerted on the outer edge portions. Therefore, the side end portions easily fit the outline of the wearer's buttocks, and fit can be further improved.

In such an underpants-shaped absorbent article, it is desirable
that a plurality of the lateral elastic members are disposed in the extending portion at an interval in the up-down direction, and
that a length by which each of the plurality of lateral elastic members extends outward in the left-right direction beyond the inclining elastic member differs.

According to such an underpants-shaped absorbent article, a force with which the outer edge portions of the back waist member is stretched inward in the left-right direction is uneven due to the stretching force of the lateral elastic members. Thus, the shape of the frills formed in the outer edge portions and the size of the recesses and protrusions are irregular. Consequently, design is improved, it is possible to give a wearer with an impression that the frills is made of soft material, and it is possible to realize soften texture during wearing.

In such an underpants-shaped absorbent article,
concerning a length by which the lateral elastic member extends on a left-right-direction one side beyond the inclining elastic member located on the left-right-direction one side of the extending portion, and
concerning a length by which the lateral elastic member extends on a left-right-direction other side beyond the inclining elastic member located on the left-right-direction other side of the extending portion,
   these lengths differ from each other.

According to such an underpants-shaped absorbent article, the lengths by which the lateral elastic members extend outward in the left-right direction beyond the inclining elastic members differs between the left outer edge portion and the right outer edge portion of the extending portion. Thus, the left and right frills are formed to be irregular in terms of shape and size. Consequently, overall design of the extending portion including the frills is improved. In addition, softness of texture is easily perceived by a wearer when the absorbent article is put on.

In such an underpants-shaped absorbent article, it is desirable that
in the underpants-shaped absorbent article in a natural state,
the extending portion includes a portion projecting outside the inclining elastic member in the left-right direction.

According to an underpants-shaped absorbent article, the portion of the extending portion projects outside the inclining elastic members in the left-right direction. Thus, the area of the extending portion appears large, and the buttocks appear wrapped. Consequently, it is possible to provide a user with a sense of security of the wearer's buttocks being reliably covered without exposing externally during wearing.

### === Embodiment ===

Hereinafter, as an underpants-shaped absorbent article according to the present invention, an embodiment will be described by presenting a pull-on disposable diaper for an adult as an example. However, the underpants-shaped absorbent article according to the present invention is not limited to a diaper and is usable as a shorts-type sanitary napkin or the like.

### Basic Configuration of Pull-on Disposable Diaper

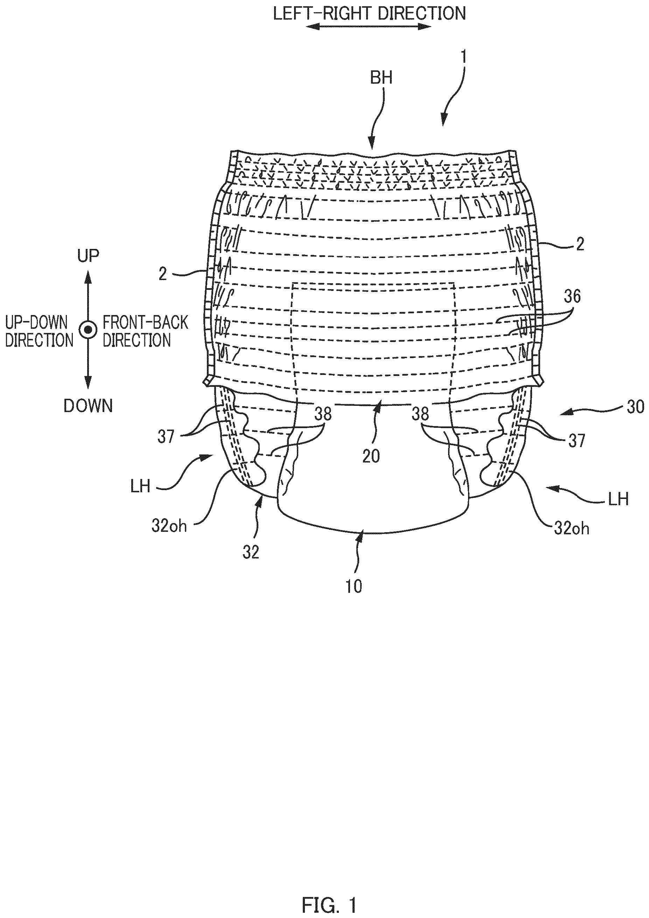

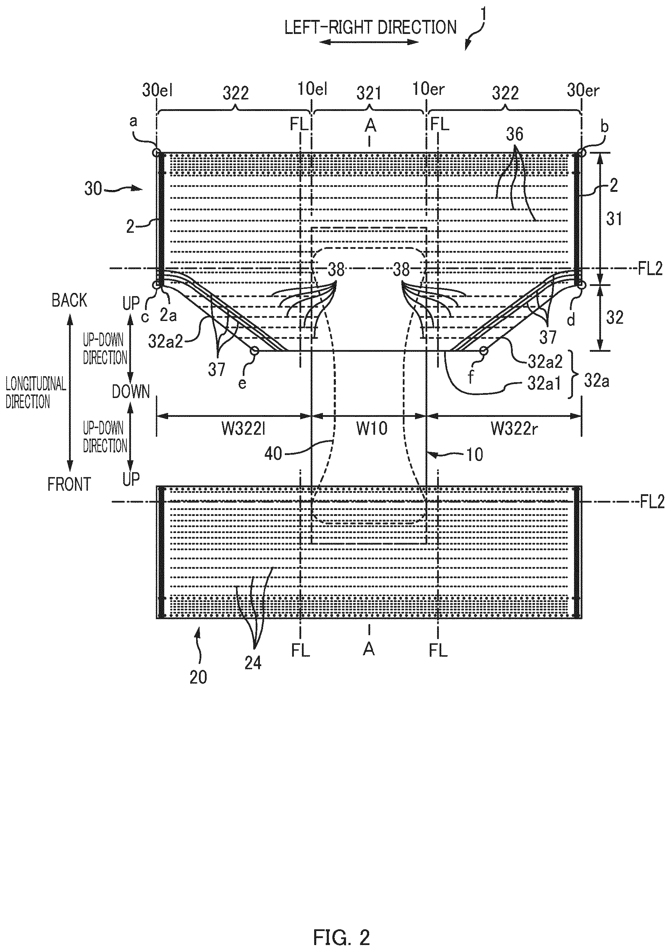

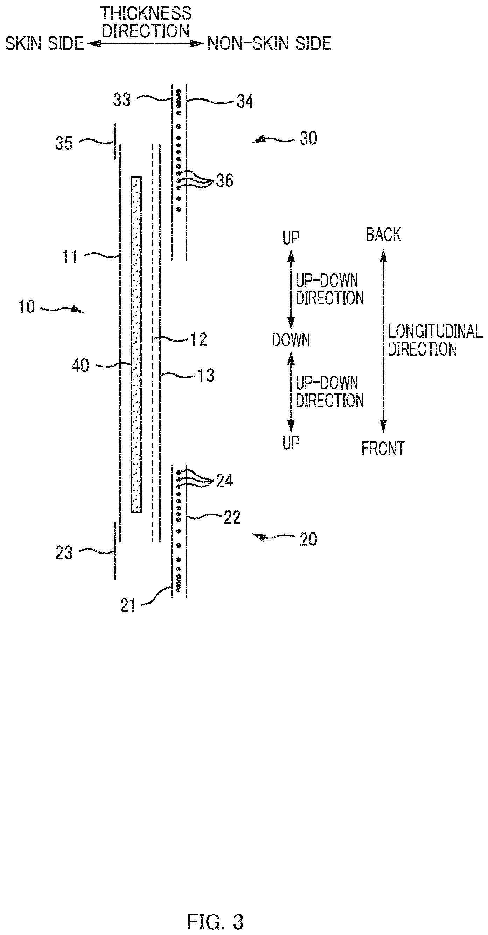

FIG. 1 is a schematic front view of a pull-on disposable diaper 1 (hereinafter "diaper") as viewed from the front side. FIG. 2 is a schematic plan view of the diaper 1 in an unfolded and stretched state as viewed from a non-skin side. FIG. 3 is a schematic cross-sectional view taken along line A-A of FIG. 2.

The stretched state of the diaper 1 is a state in which the diaper 1 is stretched to an extent that causes creases generated in the diaper 1 to be substantially visually unrecognizable. Specifically, the stretched state is a state in which the diaper 1 is stretched until the dimensions of each member (for example, the later-described skin-side sheets 21 and 31, and the like) constituting the diaper 1 coincides with the dimension of the member as a single body or becomes a length approximate thereto.

The diaper 1 in the underpants-shaped state in FIG. 1 has an up-down direction, a left-right direction, and a front-back direction that are orthogonal to each other. In the up-down direction, the side closer to the wearer's waist is the upper side, and the side closer to the wearer's crotch is the lower side. In the front-back direction, the stomach side of a wearer is the front side, and the back side of a wearer is the back side. In addition, a direction in which the members constituting the diaper 1 are layered as illustrated in FIG. 3 is the thickness direction. In the thickness direction, a side to be in contact with a wearer is the "skin side", and a side opposite thereto is the "non-skin side".

The diaper 1 is a three-piece type diaper constituted by three members: an absorbent main body 10; a front waist member 20 that covers the front side of a wearer; and a back waist member 30 that covers the back side of a wearer.

In the diaper 1 in the unfolded state in FIG. 2, the front waist member 20 and the back waist member 30 are disposed such that the longitudinal direction thereof is in the left-right direction of the diaper 1. The lateral center portion of the front waist member 20 is disposed in a longitudinal end portion of the absorbent main body 10 on one side, and the lateral center portion of the back waist member 30 is disposed in a longitudinal end portion of the absorbent main body 10 on the other side.

The diaper 1 is two-folded substantially at the longitudinal center of the absorbent main body 10. The front waist member 20 and the back waist member 30 being joined to each other in two side end portions in the left-right direction by a pair of joint portions 2, becoming in the underpants-shaped state in FIG. 1 from the unfolded state in FIG. 2. In the pull-on diaper 1, a waist opening portion BH and a pair of leg opening portions LH are formed.

The absorbent main body 10 includes: an absorbent body 40; a top sheet 11 disposed on the skin side in the thickness direction with respect to the absorbent body 40; a back sheet 12 disposed on the non-skin side in the thickness direction with respect to the absorbent body 40; and an exterior sheet 13.

The top sheet 11 is a liquid-permeable sheet. As the top sheet 11, a hydrophilic spunbond nonwoven fabric, a hydrophilic air-through nonwoven fabric, and the like are exemplified. The back sheet 12 is a liquid-impermeable sheet. As the back sheet 12, a polyethylene film and the like are exemplified. As the exterior sheet 13, a hydrophobic SMS nonwoven fabric and the like are exemplified. Although no illustration is provided, a three-dimensional gather portion erectable on the skin side may be disposed in two lateral side portions of the absorbent main body 10 by means such as folding the exterior sheet 13 toward the skin side and arranging a string-shaped elastic member, for example.

The absorbent body 40 includes an absorbent core that absorbs and holds excrement such as urine and the like. As the absorbent core, an absorbent core obtained by shaping liquid-absorbent fibers (e.g., pulp containing super absorbent polymers (SAP)) into a predetermined shape (hourglass shape in this example) is exemplified. The absorbent core may be covered with a liquid-permeable core wrapping sheet, such as tissue paper.

The front waist member 20 is a member that has a rectangular shape in the stretched state in FIG. 2. The back waist member 30 is a member that has, in the stretched state in FIG. 2, a shape constituted by a rectangular shape and a substantially reverse trapezoidal shape combined with a lower portion of the rectangular shape. The back waist member 30 has a larger planar shape than the front waist member 20.

Specifically, the back waist member 30 includes: a rectangular "waist portion 31" (portion surrounded by a, b, c, and d indicated in FIG. 2) which overlaps the joint portions 2 in the up-down direction; and a substantially reverse trapezoidal "extending portion 32" (portion surrounded by c, d, e, and f indicated in FIG. 2) which extends downward in the up-down direction with respect to the lower ends 2a of the joint portions 2. Therefore, the wearer's buttocks can be covered with the extending portion 32 even when the diaper 1 is of the three-piece type.

In addition, the extending portion 32 of the back waist member 30 includes a "center extending portion 321" which overlaps the absorbent main body 10 in the front-back direction (are overlaid the absorbent main body 10 in the thickness direction), and a pair of "outer extending portions 322" extending outward on the two lateral sides of the absorbent main body 10.

In addition, outer edges 32a of the lower portion of the extending portion 32 each include: in the center portion in the left-right direction, a "linear portion 32a1" extending in the left-right direction; and in the two end portions in the left-right direction, "inclining portions 32a2" extending downward and inclining inward in the left-right direction. The linear portion 32a1 extends outward on the two lateral sides of the absorbent main body 10.

As illustrated in FIG. 3, the front waist member 20 includes the skin-side sheet 21, a non-skin-side sheet 22, a cover sheet 23, and a plurality of waist elastic members 24 extending in the left-right direction. The plurality of waist elastic members 24 are disposed in the front waist member 20 and arranged side-by-side at intervals in the up-down direction.

The back waist member 30 includes a skin-side sheet 33, a non-skin-side sheet 34, and a cover sheet 35. In the waist portion 31 of the back waist member 30, a plurality of waist elastic members 36 are each disposed extending in the left-right direction. In the extending portion 32, a plurality of inclining elastic members 37 are each disposed inclined in the left-right direction, and a plurality of lateral elastic members 38 are each disposed extending in the left-right direction.

Specifically, the plurality of waist elastic members 36 are disposed in the waist portion 31 and arranged side-by-side at intervals in the up-down direction. The plurality (three in FIG. 2) of the inclining elastic members 37 are disposed in left-right-direction side end portions of the extending portion 32 along the inclining portions 32a2 of the outer edge 32a of the extending portion 32, and are arranged side-by-side at intervals in the left-right direction. In other words, the inclining elastic members 37 are disposed extending downward and inclining inward in the left-right direction. The plurality (five in FIG. 2) of lateral elastic members 38 are disposed in the extending portion 32 and arranged side-by-side at intervals in the up-down direction.

The skin-side sheets 21 and 33 and the non-skin-side sheets 22 and 34 are disposed on the non-skin side in the thickness direction with respect to the absorbent main body 10. The cover sheets 23 and 35 each have a shorter vertical length than the skin-side sheets 21 and 33 and the non-skin-side sheets 22 and 34. The cover sheets 23 and 35 are disposed on the skin side in the thickness direction with respect to the absorbent main body 10, and cover the longitudinal end portions of the absorbent main body 10. As these sheets 21 to 23 and 33 to 35, soft sheets of SMS nonwoven fabric or the like are exemplified.

The waist elastic members 24 and 36 and the lateral elastic members 38 are fixed with an adhesive (for example, a hot-melt adhesive or the like) between the skin-side sheet 21 (33) and the non-skin-side sheet 22 (34) so as to be stretchable in the left-right direction, while being stretched in the left-right direction. The inclining elastic members 37 are also fixed with an adhesive between the skin-side sheet 21 (33) and the non-skin-side sheet 22 (34) so as to be stretchable in a direction inclining with respect to the left-right direction, while being stretched in the inclining direction.

In the figures, illustrated are only stretchable portions (effective length portions fixed in a stretched state to the sheets) of each of the elastic members 24 and 36 to 38. In portions that are not illustrated, non-stretchable portions of each of the elastic members 24 and 36 to 38 may be present.

In FIG. 2, in the waist elastic members 36 and the lateral elastic members 38 on the back side overlapping the absorbent body 40 in the up-down direction, no stretchable portions of the elastic members 36 and 38 are present in the center portion in the left-right direction. Due to such absence of the stretchable portions of the elastic members 36 and 38 in the portions which overlaps the absorbent main body 10 in the front-back direction (are overlaid the absorbent main body 10 in the thickness direction), it is possible to suppress contraction of the absorbent main body 10 in the left-right direction.

As each of the elastic members 24 and 36 to 38, a string-shaped elastic member of rubber, spandex, or the like is exemplified. The elastic members 24 and 36 to 38 are, however, not limited thereto and may be sheet-shaped elastic members of expandable nonwoven fabric, expandable film, or the like.

### << Extending portion of back waist member >>

The diaper 1 includes the extending portion 32 in a region located on the lower side (crotch side) of the back waist member 30 in the up-down direction. During wearing of the diaper 1, the extending portion 32 covers the wearer's buttocks. In a state in which the diaper 1 is stretched in the left-right direction, the left-right-direction width of each of the outer extending portions 322, which are regions of the extending portion 32 not overlapping the absorbent main body 10, is larger than the left-right-direction width of the center extending portion 321, which is a region overlapping the absorbent main body 10. In other words, in a state in which the diaper 1 is stretched in the left-right direction as illustrated in FIG. 2, the distance W322l in the left-right direction between a left side end 10el of the absorbent main body 10 and a left side end 30el of the back waist member 30 is larger than the distance W10 between the left side end 10el and a right side end 10er of the absorbent main body 10 (W322l > W10). Similarly, the distance W322r in the left-right direction between the right side end 10er of the absorbent main body 10 and the right side end 30er of the back waist member 30 is larger than the distance W10 between the left side end 10el and the right side end 10er of the absorbent main body 10 (W322r > W10). Therefore, it is possible to reliably cover even the wide buttocks of an adult, which is a target wearer of the diaper 1.

In the outer edge 32a of the outer extending portions 322, the inclining elastic members 37 are disposed in a region extending along the inclining portions 32a2. Due to stretchability imparted by the inclining elastic members 37, the outer edge 32a easily fits along the outline of the wearer's buttocks. In other words, in the diaper 1 of the present embodiment, the inclining elastic members 37 are located on the outer side of the apex of the wearer's buttocks during wearing. It is thus possible to wrap around the wearer's buttocks while the outer extending portions 322 are not turned up even when the area of the outer extending portions 322 is large (even when the distances W322l and W322r in the left-right direction are large).

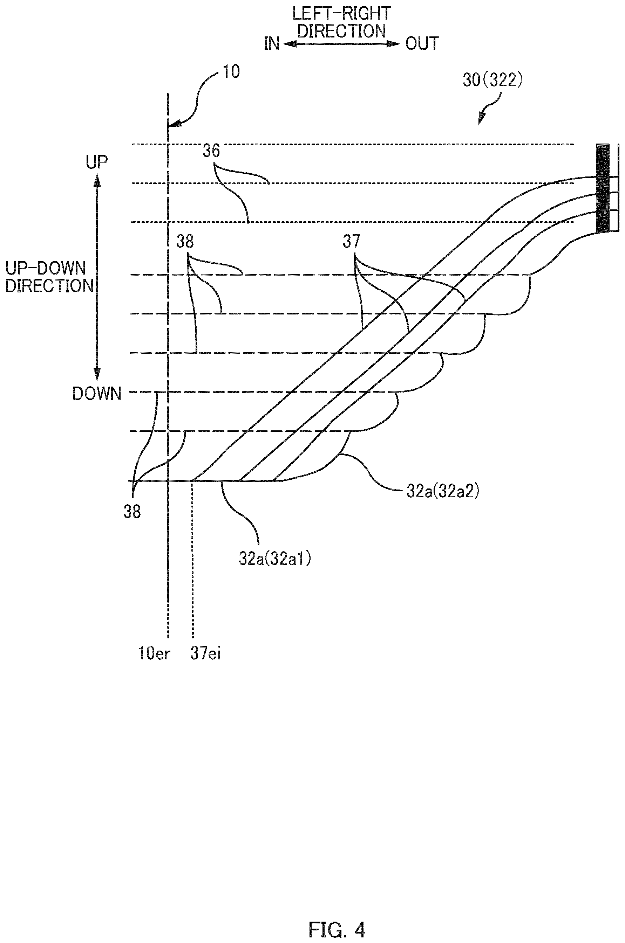

Moreover, when viewed in the front-back direction (thickness direction), the inclining elastic members 37 and the lateral elastic members 38 overlap each other (see FIG. 4) in the left-right-direction side end portions of the outer extending portions 322, and accordingly the stiffness of the outer extending portions 322 increases in these two side end portions where the two elastic members 37 and 38 overlap each other. This makes it easier to maintain a state in which the outer edge 32a of the outer extending portions 322 fits along the outline of the wearer's buttocks. Accordingly, even when the wearer moves the body, this makes it less likely for the outer extending portions 322 to turn up or displace.

In the diaper 1, due to the extending portion 32 having such a configuration, it is possible to achieve favorable fit while widely covering the wearer's buttocks.

At the extending portion 32 of the diaper 1, the lateral elastic members 38 extend outward in the left-right direction with respect to the inclining elastic members 37. FIG. 4 is an illustration of an arrangement of the inclining elastic members 37 and the lateral elastic members 38 in the outer extending portions 322 of the diaper 1. FIG. 4 illustrates an enlarged view of a portion in the vicinity of the outer edge 32a of the outer extending portion 322 on the right side in the left-right direction. As illustrated in FIG. 4, the lateral elastic members 38 include portions extending outward in the left-right direction with respect to the inclining elastic members 37.

In regions on the outer sides of the inclining elastic members 37 in the left-right direction, the skin-side sheet 33 and the non-skin-side sheet 34 of the back waist member 30 are joined to each other in the thickness direction with a hot-melt adhesive or the like. And, between the sheet 33 and 34, the extending portions of the lateral elastic members 38 are disposed. Therefore, in the outer extending portions 322, at the regions outside the inclining elastic members 37 in the left-right direction, the portions where the lateral elastic members 38 are disposed are pulled inward in the left-right direction due to an action of the stretching force of the lateral elastic members 38. Consequently, in the regions (that is, outer edge portions) of the outer extending portions 322 (extending portion 32) outside the inclining elastic members 37 in the left-right direction, the portions where the lateral elastic members 38 are disposed easily contract inward in the left-right direction. On the other hand, regions where the lateral elastic members 38 are not disposed do not easily contract inward in the left-right direction. Consequently, the outer edge 32a of the outer extending portions 322 deforms into a frill shape having recesses and protrusions in the left-right direction, as illustrated in FIG. 4. As a result of the outer edge 32a having the frill shape, the texture of the outer edge 32a during wearing of the diaper 1 is improved, making it less likely for a wearer to feel discomfort. In addition, the frill shape formed along the leg opening portions LH can provide the user a visually soft impression.

In the diaper 1 of the present embodiment, the lateral elastic members 38 extending outward in the left-right direction with respect to the inclining elastic members 37 reach the outer ends (the outer edge 32a of the outer extending portions 322) of the extending portion 32 in the left-right direction. Consequently, in regions outside the inclining elastic members 37 in the left-right direction, a larger stretching force of the lateral elastic members 38 is exerted. In addition, the stretching force makes it easier for the outer edge 32a of the outer extending portions 322 to be stretched inward in the left-right direction, increasing the size of the recesses and protrusions of the frills. Therefore, the favorable texture is realized during wearing, and large frills can improve the visual impression of a user.

As a result of exerting the stretching force by the lateral elastic members 38 on the entirety of the outer extending portions 322 in the left-right direction, the inclining elastic members 37 and the entirety of the frills in a natural state of the diaper 1 are in a state of being folded inward in the left-right direction and toward the skin side in the thickness direction, as illustrated in FIG. 1. A part of the regions of the outer extending portions 322 (extending portion 32) projects outside the inclining elastic members 37 in the left-right direction. In other words, in the natural state of the diaper 1, the extending portion 32 includes projecting portions 32oh projecting outside the inclining elastic members 37 in the left-right direction. In the external appearance of the diaper 1, the projecting portions 32oh form outer side ends of the extending portion 32 in the left-right direction (see FIG. 1). Due to the projecting portions 32oh projecting outside the inclining elastic members 37 in the left-right direction, the area of the extending portion 32 (outer extending portions 322) appears large, and the buttocks appear wrapped. Consequently, it is possible to provide a user with a sense of security of the wearer's buttocks being reliably covered without exposing externally during wearing of the diaper 1.

Here, the "natural state" of the diaper 1 is a state of the diaper 1 after being left for a predetermined period of time. For example, by stretching the front waist member 20 and the back waist member 30 of the diaper 1 outward toward two left-right-direction-side, the waist members 20 and 30 are made in an "stretched state'. Thereafter keeping the stretched state for approximately 30 seconds, and then the stretching of the diaper 1 is released, and the diaper 1 is placed on a flat surface of a desk or the like. A state after a lapse of five minutes with the diaper 1 thus placed flat on the flat surface is defined as the natural state.

As illustrated in FIG. 4, in the outer extending portions 322 (extending portion 32), a plurality of the lateral elastic members 38 are disposed at intervals in the up-down direction. Among these lateral elastic members 38, the position of the lowermost lateral elastic member 38 is higher than the position of the lower end of the inclining elastic members 37. In other words, in the up-down direction, the inclining elastic members 37 extend downward beyond the lowermost lateral elastic member 38. The inclining elastic members 37 extending downward beyond the lateral elastic members 38 makes it easier for the stretching force of the inclining elastic members 37 to be exerted on a further lower part of the outer extending portions 322. This makes it easier to suppress turning-up and displacement of the lower part of the outer extending portions 322 when the diaper 1 is put on. In addition, as a result of the inclining elastic members 37 being disposed extending downward along the outline of the wearer's buttocks, it is possible to further improve the fit around the wearer's buttocks.

In the diaper 1, the inclining elastic members 37 are disposed inclining downward in the up-down direction from the outer sides toward the inner side in the left-right direction. The inclining elastic members 37 do not reach two side ends 10el and 10er of the absorbent main body 10 in the left-right direction. In other words, a predetermined interval is provided between left-right-direction inner ends 37ei of the inclining elastic members 37 and the left-right-direction outer side end 10el (10er) of the absorbent main body 10 (see FIG. 2). The inclining elastic members 37 thus not overlaid the absorbent main body 10 in the thickness direction makes it difficult for the stretching force of the inclining elastic members 37 to be exerted on the absorbent main body 10. If the absorbent main body 10 and the inclining elastic members 37 overlap each other, there is a risk that, when the diaper 1 is put on, the absorbent main body 10 is easily displaced from the wearer's crotch as a result of the absorbent main body 10 being stretched obliquely along the inclination of the inclining elastic members 37. Moreover, as a result of the extending portion 32 being also pulled up along the absorbent main body 10, there is a case where the buttocks are less likely to be covered by the extending portion 32. In contrast, in the diaper 1 of the present embodiment, the stretching force of the inclining elastic members 37 is less likely to be exerted on the absorbent main body 10, which making it possible to suppress displacement of the absorbent main body 10 during wearing of the diaper 1. In addition, it becomes easier for the wearer's buttocks to be covered the extending portion 32. Consequently, even when the wearer moves the legs, the line of the inclining elastic members 37 disposed at the buttocks can be suppressed from being twisted and causing the extending portion 32 to be turned up.

Incidentally, the diaper 1 is packaged after being manufactured in a factory or the like and is distributed to the market in a predetermined packaged state. Specifically, when the diaper 1 is to be packaged, the front waist member 20 and the back waist member 30 of the diaper 1 in the natural state (underpants-shaped state) are folded at folding positions FL extending in the up-down direction (longitudinal direction in FIG. 2), from the outer side toward the inner side in the left-right direction. Then the front waist member 20 and the back waist member 30 are folded up at folding positions FL2 (see FIG. 2) extending in the left-right direction such that the respective heights thereof become substantially half in the up-down direction. The inclining elastic members 37 are disposed such that the inclining elastic members 37 and the folding positions FL (and FL2) do not overlap each other when the diaper 1 in a state of being thus folded up is viewed in the front-back direction. Consequently, the diaper 1 is able to be distributed to the market with the inclining elastic members 37 not folded up at the folding positions FL. If the inclining elastic members 37 overlap the folding positions FL, the inclining elastic members 37 will be distributed to the market in a state of being folded at the folding positions FL. In this case, a crease is more likely to be formed in the inclining elastic members 37, and there is a risk that the inclining elastic members 37 thus having creases become unable to impart appropriate stretchability to the outer extending portions 322 (extending portion 32). In contrast, in the diaper 1 of the present embodiment, the inclining elastic members 37 are arranged separated from the folding positions FL, making it possible to suppress a crease from being more likely to be formed, and are able to impart appropriate stretchability to the outer extending portions 322 (extending portion 32). Accordingly, due to the inclining elastic members 37, it becomes easier for the side end portions of the outer extending portions 322 to fit along the outline of the wearer's buttocks.

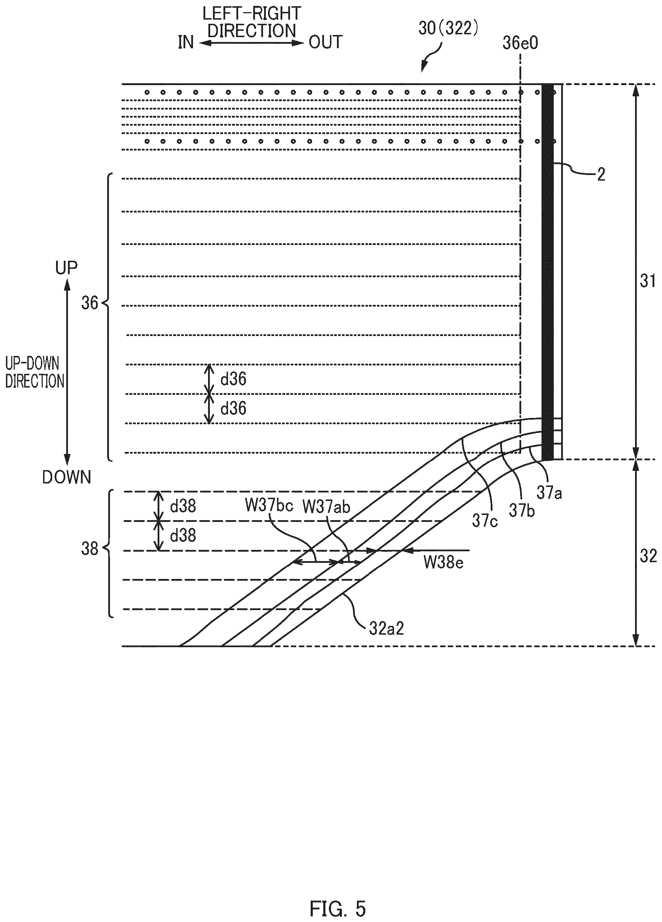

Next, FIG. 5 is an illustration of an arrangement of the waist elastic members 36 and the lateral elastic members 38 in the back waist member 30. FIG. 5 illustrates a right region of the back waist member 30 (waist portion 31 and extending portion 32) in a state of being stretched in the left-right direction and the up-down direction. As illustrated in FIG. 5, a plurality of the waist elastic members 36 are disposed in the waist portion 31 of the back waist member 30 at intervals in the up-down direction. These waist elastic members 36 are disposed inside the joint portions 2 in the left-right direction. In other words, outer side ends 36e0 of the waist elastic members 36 in the left-right direction are positioned inside the joint portions 2 in the left-right direction, and the waist elastic members 36 and the joint portions 2 do not overlap each other. Stiffness of the joint portions 2 is increased by joining means, such as welding. Thus, if the waist elastic members 36 overlap the joint portions 2, stiffness of the joint portions 2 is further increased, which easily imparts discomfort to a wearer during wearing of the diaper 1. In contrast, in the present embodiment, the waist elastic members 36 are disposed not to overlap the joint portions 2, making it possible to suppress stiffness of the joint portions 2 from being increased. It is thus possible to avoid degradation in sense of comfort in the diaper 1.

In FIG. 5, d36 denotes an interval in the up-down direction between two of the waist elastic members 36 adjacent to each other in the up-down direction, and d38 denotes an interval in the up-down direction between two of the lateral elastic members 38 adjacent to each other in the up-down direction. The interval d36 and the interval d38 are equal to each other (d36 = d38). In other words, in the diaper 1, the waist elastic members 36 and the lateral elastic members 38 are disposed at an equal pitch in the up-down direction. Enabling to dispose the elastic members of two types at an equal pitch makes it possible to simplify adjusting the positions of the elastic members 36 and 38 in a manufacturing process of the diaper 1 (back waist member 30). This facilitates manufacturing of the back waist member 30, and allows to suppress increase of manufacturing costs.

In the back waist member 30, the interval d38 of the lateral elastic members 38 in the up-down direction may be larger than the interval d36 of the waist elastic members 36 in the up-down direction (d38 > d36). In this case, the pitch of the lateral elastic members 38 in the up-down direction increases in the extending portion 32 (outer extending portions 322) of the back waist member 30, and therefore, the stretching force which the lateral elastic members 38 exerts on a unit area of the extending portion 32 decreases. Accordingly, during wearing of the diaper 1, the fastening force by the outer extending portions 322 that cover the wearer's buttocks decreases. This prevents the force for fastening at the buttocks from being excessively strong, and it is possible to suppress degradation in sense of comfort of the diaper 1 and to give appropriate fitting to the wearer.

Conversely, in the back waist member 30, the interval d38 of the lateral elastic members 38 in the up-down direction may be smaller than the interval d36 of the waist elastic members 36 in the up-down direction (d38 < d36). While it is possible to decrease the fastening force when increasing the pitch of the lateral elastic members 38 in the up-down direction as described above, there is a case in which the outer extending portions 322 separates away from the wearer's buttocks or turning-up thereof occurs, for example, when the wearer's body (buttocks) is small. In this case, decreasing the pitch of the lateral elastic members 38 in the up-down direction makes it easier for the stretching force by the lateral elastic members 38 to be exerted on the entirety of the outer extending portions 322, and this makes it easier for the outer extending portions 322 to fit along the wearer's buttocks in surface-to-surface contact. Consequently, it is more likely to suppress separating away from the outer extending portions 322 during wearing of the diaper 1.

Thus, optimal fit can be achieved in the diaper 1 by adjusting the up-down-direction pitches of the waist elastic members 36 and the lateral elastic members 38 in accordance with the target wearer's body (buttocks) size, a usage and the like. For example, in a usage such that a wearer of the diaper 1 lies in a bed for a prolonged period, if the diaper 1 in which the pitch of the lateral elastic members 38 is wide is selected, excessive fastening at the buttocks is suppressed and it is possible to prevent the wearer from feeling discomfort. In contrast, in a usage such that a wearer lives an ordinary life in a state of wearing the diaper 1, if the diaper 1 in which the pitch of the lateral elastic members 38 is narrow is selected, displacement and turning-up of the outer extending portions 322 is suppressed even when the wearer moves the body, and it is possible for the wearer to feel favorable fit.

In the diaper 1, the plurality of inclining elastic members 37 are disposed with gaps therebetween in the left-right direction, but each gap between each pair of the inclining elastic members 37 adjacent to each other in the left-right direction is not constant. In FIG. 5, three inclining elastic members 37a, 37b, and 37c are disposed from the outer side toward the inner side in the left-right direction. The gap W37ab in the left-right direction between the inclining elastic members 37a and 37b is not constant, and the gap W37bc in the left-right direction between the inclining elastic members 37b and 37c is also not constant. In other words, the inclining elastic members 37a to 37c are disposed along the inclining portions 32a2 (outer edge 32a) of the outer extending portions 322, but are not disposed exactly parallel to the inclining portions 32a2. That is, the inclining elastic members 37a to 37c are disposed to meander with respect to the inclining direction of the inclining portions 32a2.

In the diaper 1, the maximum value of the gap W37ab in FIG. 5 is smaller than the minimum value of the gap w37bc. In other words, of the left-right-direction gaps between the inclining elastic members 37 adjacent to each other in the left-right direction, the maximum value of the outermost gap is smaller than the minimum value of the other gaps. Due to the gap between the inclining elastic members 37a and 37b arranged nearest to outer side ends (outer edge portions) of the inclining portions 32a2 in the left-right direction thus being narrow, the stretching force of the inclining elastic members 37 (37a and 37b) concentrates and is easily exerted on the side end portion. In other words, a contraction force exerted on a unit area of the outer edge portions of the extending portion 32 increases. Thus, the outer edge portions easily fit the outline of the wearer's buttocks during wearing of the diaper 1, and fit can be further improved.

In the diaper 1, due to the inclining elastic members 37a disposed outermost in the left-right direction being disposed to meander with respect to the inclining direction of the inclining portions 32a2, the distance in the left-right direction between the inclining elastic members 37a and the inclining portions 32a2 (outer edge 32a of the outer extending portions 322) is also not constant. Thus, the length W38e of the lateral elastic members 38 extending outward in the left-right direction beyond the inclining elastic members 37 is also not constant. In other words, the length by which each of the plurality of lateral elastic members 38 extends outward in the left-right direction beyond the inclining elastic members 37 differs. Therefore, due to the stretching force of the lateral elastic members 38, the force with which the outer edge portions of the inclining portions 32a2 are stretched inward in the left-right direction is uneven. The shape of frills formed in the outer edge portions and the size of the recesses and protrusions are thus irregular. Consequently, design of the outer extending portions 322 is improved. Moreover, it is possible to give a wearer with an impression that the frills is made of soft material, and it is possible to realize soften texture during wearing.

### Modified examples of Diaper 1

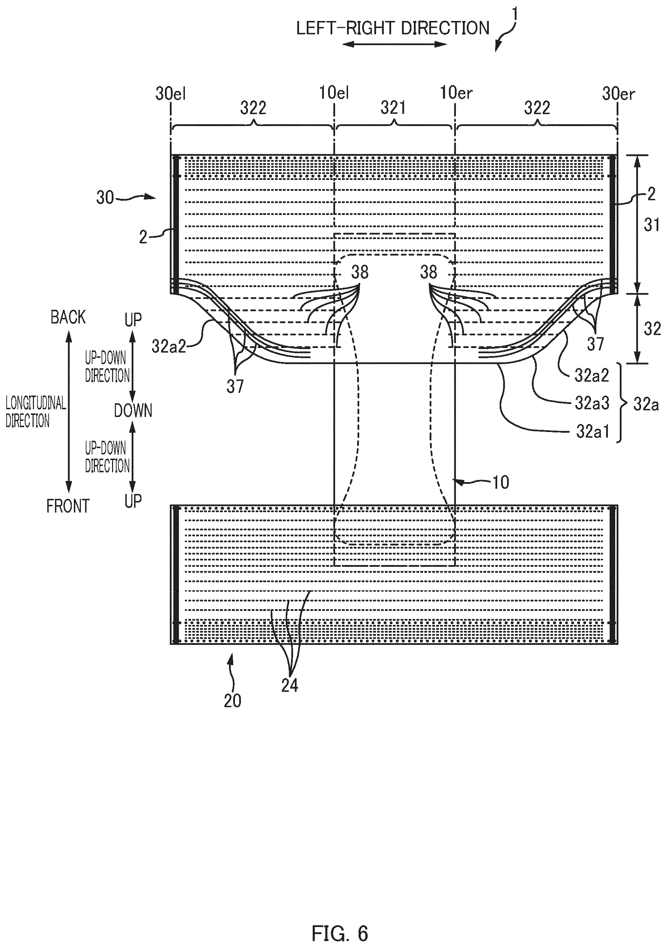

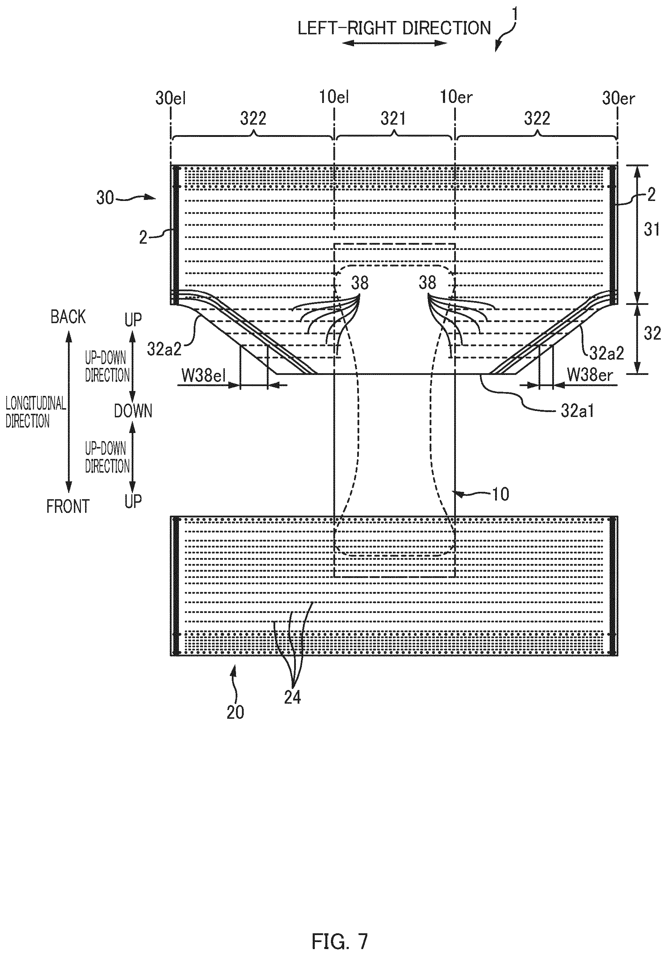

The diaper 1 can be modified as in a first modified example and a second modified example described below. FIG. 6 is a schematic plan view of the diaper 1 of the first modified example in an unfolded and stretched state as viewed from the skin side. FIG. 7 is a schematic plan view of the diaper 1 of the second modified example in an unfolded and stretched state as viewed from the skin side.

In the diaper 1 of the first modified example, the shape of the back waist member 30 differs from that in the aforementioned embodiment. Specifically, as illustrated in FIG. 6, at the outer edge 32a of the back waist member 30, the linear portion 32a1 and the inclining portions 32a2 are continuously connected to each other by curved portions 32a3. In other words, on the lower side of the back waist member 30 (outer extending portions 322), part of the outer edge 32a is formed in a curved manner. The other configurations are identical to those of the aforementioned diaper 1, and thus, detailed description thereof is omitted.

In the modified example 1, no corner is formed at the outer edge 32a of the outer extending portions 322. Thus, the outer edge 32a of the outer extending portions 322 is in smooth contact with the wearer's buttocks when the diaper 1 is put on and can impart a soft texture to the wearer. In addition, the inclining elastic members 37 are disposed in a curved manner along the curved portions 32a3, and a part of the inclining elastic members 37 extend parallel to the left-right direction along the linear portion 32a1. In other words, the stretchability in the left-right direction is exerted in the lower end portion (linear portion 32a1) of the outer edge 32a of the outer extending portions 322. Consequently, during wearing of the diaper 1, the outer extending portions 322 easily follow the outline of the wearer's buttocks, and fitting around the buttocks can be further improved.

In the diaper 1 of the second modified example, the arrangement of the inclining elastic members 37 is left-right asymmetric. Specifically, the inclining elastic members 37 disposed on the left-right-direction one side of the back waist member 30 are positioned further inside (that is, closer to the absorbent main body 10) in the left-right direction than the inclining elastic members 37 disposed on the left-right-direction other side are positioned. The other configurations are identical to those of the aforementioned diaper 1.

In the example in FIG. 7, when the length by which the lateral elastic members 38 extend on the right side (outer side in the left-right direction) beyond the inclining elastic members 37 in the outer extending portion 322 on the right side of the absorbent main body 10 is defined as a length W38er, and the length by which the lateral elastic members 38 extend leftward (outward in the left-right direction) beyond the inclining elastic members 37 in the outer extending portion 322 located on the left side of the absorbent main body 10 is defined as a length W38el, the length W38er and the length W38el differ from each other. In other words, the length by which the lateral elastic members 38 extend outward in the left-right direction beyond the inclining elastic members 37 differs between the left outer edge portion and the right outer edge portion of the outer extending portions 322. Consequently, left and right frills are formed to have irregular shapes and sizes. Therefore, overall design of the outer extending portions 322 is improved. In addition, it is possible to cause a wearer to perceive soft texture during wearing.

### Other embodiments

Although the embodiment of the present disclosure have been described hereinabove, the above embodiment of the present disclosure is simply to facilitate understanding of the present disclosure and are not in any way to be construed as limiting the present disclosure. The present disclosure may variously be changed or altered and encompass equivalents thereof.

In the aforementioned embodiment, nonwoven fabric is presented as an example of the material of each of the exterior sheet 13, the front waist member 20, and the back waist member 30; however, the material thereof is in no way limited to nonwoven fabric. For example, woven fabric or a sheet material other than woven fabric may be employed. Incidentally, the exterior sheet 13 may be omitted. In this case, the back sheet 12 forms the exterior of the absorbent main body 10.

In the aforementioned embodiment, the waist elastic members 36 and the lateral elastic members 38 are cut in the left-right-direction center region of the back waist member 30, namely, in a part of the region of the back waist member 30 overlapping the absorbent main body 10 so that stretchability imparted by the elastic members 36 and 38 is not exerted on the center region; however, such a configuration is a non-limiting feature. For example, the following configuration is also acceptable: the elastic members 36 and 38 are disposed continuously in the left-right-direction center region and thereby impart stretchability to the region. Conversely, in the front waist member 20, although the waist elastic members 24 are disposed continuously in the left-right direction in the left-right-direction center region, the waist elastic members 24 may have a discontinuous portion in the center region.

### [Reference Signs List]

1 diaper (underpants-shaped absorbent article)
2 joint portion
10 absorbent main body
11 top sheet, 12 back sheet, 13 exterior sheet
20 front waist member
21 skin-side sheet, 22 non-skin-side sheet, 23 cover sheet, 24 waist elastic member
30 back waist member
31 waist portion
32 extending portion, 321 center extending portion, 322 outer extending portion
32a outer edge, 32a1 linear portion, 32a2 inclining portion, 32a3 curved portion
32oh projecting portion
33 skin-side sheet, 34 non-skin-side sheet, 35 cover sheet
36 waist elastic member
37 inclining elastic member, 37a, 37b, 37c inclining elastic member, 38 lateral elastic member
40 absorbent body
BH waist opening portion, LH leg opening portion

## Claims

1. An underpants-shaped absorbent article having an up-down direction, a left-right direction, and a front-back direction that are orthogonal to each other,
the underpants-shaped absorbent article comprising:
an absorbent main body (10);
a front waist member (20); and
a back waist member (30),
the front waist member (20) and the back waist member (30) being joined to each other in two side end portions in the left-right direction by a pair of joint portions (2),
the back waist member (30) including an extending portion (32) that extends downward with respect to the joint portions (2),
the extending portion (32) including
an inclining elastic member (37) being inclined with respect to the left-right direction, in a left-right-direction one side end portion of the extending portion (32),
an inclining elastic member (37) being inclined with respect to the left-right direction, in a left-right-direction other side end portion of the extending portion (32),
and a lateral elastic member (38) extending in the left-right direction,
when viewed in the front-back direction, the inclining elastic members (37) and the lateral elastic member (38) having an overlapping portion where the inclining elastic member (37) and the lateral elastic member (38) overlap each other, wherein
in a state in which the underpants-shaped absorbent article is stretched in the left-right direction, a length from a side end of the absorbent main body (10) on one side in the left-right direction to a side end of the back waist member (30) on the one side in the left-right direction is longer than a length of the absorbent main body (10) in the left-right direction, and wherein
the arrangement of the inclining elastic members is left-right asymmetric, the inclining elastic member disposed on the left-right direction one side being closer to the absorbent main body than the inclining elastic member disposed on the left-right direction other side, such that
concerning a length by which the lateral elastic member (38) extends on a left side beyond the inclining elastic member (37) in the extending portion located on the left side of the absorbent main body (10), and
concerning a length by which the lateral elastic member (38) extends on a right side beyond the inclining elastic member (37) in the extending portion located on the right side of the absorbent main body (10),
these lengths differ from each other.

2. The underpants-shaped absorbent article according to claim 1, wherein
the lateral elastic member (38) extends outward in the left-right direction with respect to the inclining elastic member (37).

3. The underpants-shaped absorbent article according to claim 2, wherein
the lateral elastic member (38) reaches a left-right-direction side end of the extending portion (32).

4. The underpants-shaped absorbent article according to any one of claims 1 to 3, wherein
a plurality of the lateral elastic members (38) are disposed in the extending portion (32) at an interval in the up-down direction, and
a lower end of the inclining elastic member (37) extends downward beyond a lowermost one of the plurality of lateral elastic members (38).

5. The underpants-shaped absorbent article according to any one of claims 1 to 4, wherein
the inclining elastic member (37) inclines downward in the up-down direction from an outer side toward an inner side in the left-right direction, and
the inclining elastic member (37) does not reach a side end of the absorbent main body (10) in the left-right direction.

6. The underpants-shaped absorbent article according to any one of claims 1 to 5, wherein
in a packaged state of the underpants-shaped absorbent article,
the front waist member (20) and the back waist member (30) are folded at a folding position extending in the up-down direction, toward an inner side in the left-right direction, and
the inclining elastic member (37) and the folding position do not overlap each other when viewed in the front-back direction.

7. The underpants-shaped absorbent article according to any one of claims 1 to 6, wherein
the back waist member (30) includes a waist portion (31) on an upper side of the extending portion (32),
the waist portion (31) includes a waist elastic member (36) extending in the left-right direction, and
the waist elastic member (36) is disposed inside the joint portion (2) in the left-right direction such that the waist elastic member (36) and the joint portion (2) do not overlap each other.

8. The underpants-shaped absorbent article according to claim 7, wherein
a plurality of the waist elastic members (36) are disposed in the waist portion (31) at an interval in the up-down direction,
a plurality of the lateral elastic members (38) are disposed in the extending portion (32) at an interval in the up-down direction, and
an interval in the up-down direction between the waist elastic members (36) adjacent in the up-down direction is equal to an interval in the up-down direction between the lateral elastic members (38) adjacent in the up-down direction.

9. The underpants-shaped absorbent article according to claim 7, wherein
a plurality of the waist elastic members (36) are disposed in the waist portion (31) at an interval in the up-down direction,
a plurality of the lateral elastic members (38) are disposed in the extending portion (32) at an interval in the up-down direction, and
an interval in the up-down direction between the lateral elastic members (38) adjacent in the up-down direction is larger than an interval in the up-down direction between the waist elastic members (36) adjacent in the up-down direction.

10. The underpants-shaped absorbent article according to claim 7, wherein
a plurality of the waist elastic members (36) are disposed in the waist portion (31) at an interval in the up-down direction,
a plurality of the lateral elastic members (38) are disposed in the extending portion (32) at an interval in the up-down direction, and
an interval in the up-down direction between the lateral elastic members (38) adjacent in the up-down direction is smaller than an interval in the up-down direction between the waist elastic members (36) adjacent in the up-down direction.

11. The underpants-shaped absorbent article according to any one of claims 1 to 10, wherein
a plurality of the inclining elastic members (37) are disposed in the extending portion (32) at a gap in the left-right direction,
a maximum value of a left-right-direction gap between a left-right-direction outermost pair of the inclining elastic members (37) adjacent to each other in the left-right direction
is smaller than
a minimum value of a left-right-direction gap between a pair of the inclining elastic members (37) adjacent to each other in the left-right direction located inside the outermost pair in the left-right direction.

12. The underpants-shaped absorbent article according to any one of claims 1 to 11, wherein
a plurality of the lateral elastic members (38) are disposed in the extending portion (32) at an interval in the up-down direction, and
a length by which each of the plurality of lateral elastic members (38) extends outward in the left-right direction beyond the inclining elastic member (37) differs.

13. The underpants-shaped absorbent article according to any one of claims 1 to 12, wherein
in the underpants-shaped absorbent article in a natural state,
the extending portion (32) includes a portion projecting outside the inclining elastic member (37) in the left-right direction.

## Patentansprüche

1. Unterhosen-förmiger absorbierender Artikel, der eine Oben-Unten-Richtung, eine Links-Rechts-Richtung und eine Vorn-Hinten-Richtung aufweist, die rechtwinklig zueinander vorliegen,
wobei der Unterhosen-förmige absorbierende Artikel Folgendes umfasst:
einen absorbierenden Hauptkörper (10);
ein vorderes Taillenelement (20); und
ein hinteres Taillenelement (30),
wobei das vordere Taillenelement (20) und das hintere Taillenelement (30) in zwei Seitenendteilen in der Links-Rechts-Richtung durch ein Paar von Verbindungsteilen (2) miteinander verbunden sind,
das hintere Taillenelement (30) einen sich erstreckenden Teil (32) einschließt, der sich nach unten in Bezug auf die Verbindungsteile (2) erstreckt,
wobei der sich erstreckende Teil (32) Folgendes einschließt:
ein sich neigendes elastisches Element (37), das in Bezug auf die Links-Rechts-Richtung in einem Seitenendteil in Links-Rechts-Richtung des sich erstreckenden Teils (32) geneigt ist,
ein sich neigendes elastisches Element (37), das in Bezug auf die Links-Rechts-Richtung in einem anderen Seitenendteil in Links-Rechts-Richtung des sich erstreckenden Teils (32) geneigt ist,
und ein laterales elastisches Element (38), das sich in der Links-Rechts-Richtung erstreckt,
wobei bei Betrachtung in der Vorn-Hinten-Richtung die sich neigenden elastischen Elemente (37) und das laterale elastische Element (38) einen überlappenden Teil aufweisen, wo das sich neigende elastische Element (37) und das laterale elastische Element (38) miteinander überlappen, wobei
in einem Zustand, in dem der Unterhosen-förmige absorbierende Artikel in der Links-Rechts-Richtung gedehnt ist, eine Länge von einem Seitenende des absorbierenden Hauptkörpers (10) auf einer Seite in der Links-Rechts-Richtung zu einem Seitenende des hinteren Taillenelements (30) auf der einen Seite in der Links-Rechts-Richtung länger ist als eine Länge des absorbierenden Hauptkörpers (10) in der Links-Rechts-Richtung, und wobei
die Anordnung der sich neigenden elastischen Elemente in der Links-Rechts-Richtung asymmetrisch ist, wobei das sich neigende elastische Element, das an einer Seite in der Links-Rechts-Richtung angeordnet ist, näher zu dem absorbierenden Hauptkörper vorliegt als das sich neigende elastische Element, das an der anderen Seite in der Links-Rechts-Richtung angeordnet ist, sodass
hinsichtlich einer Länge, um die sich das laterale elastische Element (38) auf einer linken Seite über das sich neigende elastische Element (37) in dem sich erstreckenden Teil hinaus, das sich auf der linken Seite des absorbierenden Hauptkörpers (10) befindet, erstreckt, und
hinsichtlich einer Länge, um die sich das laterale elastische Element (38) auf einer rechten Seite über das sich neigende elastische Element (37) in dem sich erstreckenden Teil hinaus, das sich auf der rechten Seite des absorbierenden Hauptkörpers (10) befindet, erstreckt,
sich diese Längen voneinander unterscheiden.

2. Unterhosen-förmiger absorbierender Artikel nach Anspruch 1, wobei
sich das laterale elastische Element (38) nach außen in der Links-Rechts-Richtung in Bezug auf das sich neigende elastische Element (37) erstreckt.

3. Unterhosen-förmiger absorbierender Artikel nach Anspruch 2, wobei
das laterale elastische Element (38) ein Seitenende in Links-Rechts-Richtung des sich erstreckenden Teils (32) erreicht.

4. Unterhosen-förmiger absorbierender Artikel nach einem der Ansprüche 1 bis 3, wobei
eine Vielzahl der lateralen elastischen Elemente (38) in dem sich erstreckenden Teil (32) in einem Abstand in der Oben-Unten-Richtung angeordnet ist und
sich ein unteres Ende des sich neigenden elastischen Elements (37) nach unten über ein unterstes der Vielzahl von lateralen elastischen Elementen (38) hinaus erstreckt.

5. Unterhosen-förmiger absorbierender Artikel nach einem der Ansprüche 1 bis 4, wobei
sich das sich neigende elastische Element (37) nach unten in der Oben-Unten-Richtung von einer äußeren Seite in Richtung einer inneren Seite in der Links-Rechts-Richtung neigt und
das sich neigende elastische Element (37) kein Seitenende des absorbierenden Hauptkörpers (10) in der Links-Rechts-Richtung erreicht.

6. Unterhosen-förmiger absorbierender Artikel nach einem der Ansprüche 1 bis 5, wobei
in einem gepackten Zustand des Unterhosen-förmigen absorbierenden Artikels
das vordere Taillenelement (20) und das hintere Taillenelement (30) an einer Faltposition, die sich in der Oben-Unten-Richtung erstreckt, zu einer inneren Seite in der Links-Rechts-Richtung gefaltet sind, und
das sich neigende elastische Element (37) und die Faltposition, bei Betrachtung in der Vorn-Hinten-Richtung, nicht miteinander überlappen.

7. Unterhosen-förmiger absorbierender Artikel nach einem der Ansprüche 1 bis 6, wobei
das hintere Taillenelement (30) einen Taillenteil (31) an einer oberen Seite des sich erstreckenden Teils (32) einschließt,
der Taillenteil (31) ein elastisches Taillenelement (36) einschließt, das sich in der Links-Rechts-Richtung erstreckt, und
das elastische Taillenelement (36) innen von dem Verbindungsteil (2) in der Links-Rechts-Richtung derart angeordnet ist, dass das elastische Taillenelement (36) und der Verbindungsteil (2) nicht miteinander überlappen.

8. Unterhosen-förmiger absorbierender Artikel nach Anspruch 7, wobei
eine Vielzahl der elastischen Taillenelemente (36) in dem Taillenteil (31) in einem Abstand in der Oben-Unten-Richtung angeordnet ist,
eine Vielzahl der lateralen elastischen Elemente (38) in dem sich erstreckenden Teil (32) in einem Abstand in der Oben-Unten-Richtung angeordnet ist und
ein Abstand in der Oben-Unten-Richtung zwischen den elastischen Taillenelementen (36), benachbart in der Oben-Unten-Richtung, gleich einem Abstand in der Oben-Unten-Richtung zwischen den lateralen elastischen Elementen (38), benachbart in der Oben-Unten-Richtung, ist.

9. Unterhosen-förmiger absorbierender Artikel nach Anspruch 7, wobei
eine Vielzahl der elastischen Taillenelemente (36) in dem Taillenteil (31) in einem Abstand in der Oben-Unten-Richtung angeordnet ist,
eine Vielzahl der lateralen elastischen Elemente (38) in dem sich erstreckenden Teil (32) in einem Abstand in der Oben-Unten-Richtung angeordnet ist und
ein Abstand in der Oben-Unten-Richtung zwischen den lateralen elastischen Elementen (38), benachbart in der Oben-Unten-Richtung, größer ist als ein Abstand in der Oben-Unten-Richtung zwischen den elastischen Taillenelementen (36), benachbart in der Oben-Unten-Richtung.

10. Unterhosen-förmiger absorbierender Artikel nach Anspruch 7, wobei
eine Vielzahl der elastischen Taillenelemente (36) in dem Taillenteil (31) in einem Abstand in der Oben-Unten-Richtung angeordnet ist,
eine Vielzahl der lateralen elastischen Elemente (38) in dem sich erstreckenden Teil (32) in einem Abstand in der Oben-Unten-Richtung angeordnet ist und
ein Abstand in der Oben-Unten-Richtung zwischen den lateralen elastischen Elementen (38), benachbart in der Oben-Unten-Richtung, kleiner ist als ein Abstand in der Oben-Unten-Richtung zwischen den elastischen Taillenelementen (36), benachbart in der Oben-Unten-Richtung.

11. Unterhosen-förmiger absorbierender Artikel nach einem der Ansprüche 1 bis 10, wobei
eine Vielzahl der sich neigenden elastischen Elemente (37) in dem sich erstreckenden Teil (32) in einem Abstand in der Links-Rechts-Richtung angeordnet ist,
ein maximaler Wert eines Abstands in Links-Rechts-Richtung zwischen einem äußersten Paar in Links-Rechts-Richtung der sich neigenden elastischen Elemente (37), benachbart zueinander in der Links-Rechts-Richtung,
kleiner ist als
ein minimaler Wert eines Abstands in Links-Rechts-Richtung zwischen einem Paar der sich neigenden elastischen Elemente (37), benachbart zueinander in der Links-Rechts-Richtung, das sich innen von dem äußersten Paar in der Links-Rechts-Richtung befindet.

12. Unterhosen-förmiger absorbierender Artikel nach einem der Ansprüche 1 bis 11, wobei
eine Vielzahl der lateralen elastischen Elemente (38) in dem sich erstreckenden Teil (32) in einem Abstand in der Oben-Unten-Richtung angeordnet ist und
eine Länge, um die sich jedes der Vielzahl von lateralen elastischen Elementen (38) nach außen in der Links-Rechts-Richtung über das sich neigende elastische Element (37) hinaus erstreckt, variiert.

13. Unterhosen-förmiger absorbierender Artikel nach einem der Ansprüche 1 bis 12, wobei
in dem Unterhosen-förmigen absorbierenden Artikel in einem natürlichen Zustand
der sich erstreckende Teil (32) einen Teil einschließt, der außerhalb des sich neigenden elastischen Elements (37) in der Links-Rechts-Richtung übersteht.

## Revendications

1. Article absorbant en forme de culotte ayant une direction allant de haut en bas, une direction allant de gauche à droite et une direction allant d'avant en arrière qui sont orthogonales les unes par rapport aux autres,
l'article absorbant en forme de culotte comportant :
un corps principal absorbant (10) ;
un élément avant au niveau de la taille (20) ; et
un élément arrière au niveau de la taille (30),
l'élément avant au niveau de la taille (20) et l'élément arrière au niveau de la taille (30) étant reliés l'un à l'autre en deux parties d'extrémité latérales dans la direction allant de gauche à droite par une paire de parties d'assemblage (2),
l'élément arrière au niveau de la taille (30) comprenant une partie d'extension (32) qui s'étend vers le bas par rapport aux parties d'assemblage (2),
la partie d'extension (32) comprenant
un élément élastique inclinable (37) qui est incliné par rapport à la direction allant de gauche à droite, dans une partie d'extrémité latérale dans une direction allant de gauche à droite de la partie d'extension (32),
un élément élastique inclinable (37) qui est incliné par rapport à la direction allant de gauche à droite, dans une autre partie d'extrémité latérale dans une direction allant de gauche à droite de la partie d'extension (32),
et un élément élastique latéral (38) s'étendant dans la direction allant de gauche à droite,
vus dans la direction allant d'avant en arrière, les éléments élastiques inclinables (37) et l'élément élastique latéral (38) ayant une partie de chevauchement où l'élément élastique inclinable (37) et l'élément élastique latéral (38) se chevauchent l'un par rapport à l'autre, dans lequel
dans un état dans lequel l'article absorbant en forme de culotte est étiré dans la direction allant de gauche à droite, une longueur allant d'une extrémité latérale du corps principal absorbant (10) sur un côté dans la direction allant de gauche à droite à une extrémité latérale de l'élément arrière au niveau de la taille (30) sur ledit un côté dans la direction allant de gauche à droite est plus longue qu'une longueur du corps principal absorbant (10) dans la direction allant de gauche à droite, et dans lequel
l'agencement des éléments élastiques inclinables est asymétrique de gauche à droite, l'élément élastique inclinable disposé sur un côté dans la direction allant de gauche à droite étant plus proche du corps principal absorbant que l'élément élastique inclinable disposé sur l'autre côté dans la direction allant de gauche à droite, de telle sorte que
en ce qui concerne une longueur sur laquelle l'élément élastique latéral (38) s'étend sur un côté gauche au-delà de l'élément élastique inclinable (37) dans la partie d'extension située sur le côté gauche du corps principal absorbant (10), et
en ce qui concerne la longueur sur laquelle l'élément élastique latéral (38) s'étend sur un côté droit au-delà de l'élément élastique inclinable (37) dans la partie d'extension située sur le côté droit du corps principal absorbant (10),
ces longueurs diffèrent l'une par rapport à l'autre.

2. Article absorbant en forme de culotte selon la revendication 1, dans lequel
l'élément élastique latéral (38) s'étend vers l'extérieur dans la direction allant de gauche à droite par rapport à l'élément élastique inclinable (37).

3. Article absorbant en forme de culotte selon la revendication 2, dans lequel
l'élément élastique latéral (38) atteint une extrémité latérale dans la direction allant de gauche à droite de la partie d'extension (32).

4. Article absorbant en forme de culotte selon l'une quelconque des revendications 1 à 3, dans lequel
une pluralité des éléments élastiques latéraux (38) sont disposés dans la partie d'extension (32) selon un intervalle dans la direction allant de haut en bas, et
une extrémité inférieure de l'élément élastique inclinable (37) s'étend vers le bas au-delà d'un élément élastique latéral se trouvant le plus en bas de la pluralité d'éléments élastiques latéraux (38).

5. Article absorbant en forme de culotte selon l'une quelconque des revendications 1 à 4, dans lequel
l'élément élastique inclinable (37) s'incline vers le bas dans la direction allant de haut en bas depuis un côté extérieur vers un côté intérieur dans la direction allant de gauche à droite, et
l'élément élastique inclinable (37) n'atteint pas une extrémité latérale du corps principal absorbant (10) dans la direction allant de gauche à droite.

6. Article absorbant en forme de culotte selon l'une quelconque des revendications 1 à 5, dans lequel
dans un état emballé de l'article absorbant en forme de culotte,
l'élément avant au niveau de la taille (20) et l'élément arrière au niveau de la taille (30) sont pliés au niveau d'une position de pliage s'étendant dans la direction allant de haut en bas, vers un côté intérieur dans la direction allant de gauche à droite, et
l'élément élastique inclinable (37) et la position de pliage ne se chevauchent pas l'un par rapport à l'autre quand ils sont vus dans la direction allant d'avant en arrière.

7. Article absorbant en forme de culotte selon l'une quelconque des revendications 1 à 6, dans lequel
l'élément arrière au niveau de la taille (30) comprend une partie au niveau de la taille (31) sur un côté supérieur de la partie d'extension (32),
la partie au niveau de la taille (31) comprend un élément élastique au niveau de la taille (36) s'étendant dans la direction allant de gauche à droite, et
l'élément élastique au niveau de la taille (36) est disposé à l'intérieur de la partie d'assemblage (2) dans la direction allant de gauche à droite de telle sorte que l'élément élastique au niveau de la taille (36) et la partie d'assemblage (2) ne se chevauchent pas l'un par rapport à l'autre.

8. Article absorbant en forme de culotte selon la revendication 7, dans lequel
une pluralité des éléments élastiques au niveau de la taille (36) sont disposés dans la partie au niveau de la taille (31) selon un intervalle dans la direction allant de haut en bas,
une pluralité des éléments élastiques latéraux (38) sont disposés dans la partie d'extension (32) selon un intervalle dans la direction allant de haut en bas, et
un intervalle dans la direction allant de haut en bas entre les éléments élastiques au niveau de la taille (36) adjacents dans la direction allant de haut en bas est égal à un intervalle dans la direction allant de haut en bas entre les éléments élastiques latéraux (38) adjacents dans la direction allant de haut en bas.

9. Article absorbant en forme de culotte selon la revendication 7, dans lequel
une pluralité des éléments élastiques au niveau de la taille (36) sont disposés dans la partie au niveau de la taille (31) selon un intervalle dans la direction allant de haut en bas,
une pluralité des éléments élastiques latéraux (38) sont disposés dans la partie d'extension (32) selon un intervalle dans la direction allant de haut en bas, et
un intervalle dans la direction allant de haut en bas entre les éléments élastiques latéraux (38) adjacents dans la direction allant de haut en bas est plus grand qu'un intervalle dans la direction allant de haut en bas entre les éléments élastiques au niveau de la taille (36) adjacents dans la direction allant de haut en bas.

10. Article absorbant en forme de culotte selon la revendication 7, dans lequel
une pluralité des éléments élastiques au niveau de la taille (36) sont disposés dans la partie au niveau de la taille (31) selon un intervalle dans la direction allant de haut en bas,
une pluralité des éléments élastiques latéraux (38) sont disposés dans la partie d'extension (32) selon un intervalle dans la direction allant de haut en bas, et
un intervalle dans la direction allant de haut en bas entre les éléments élastiques latéraux (38) adjacents dans la direction allant de haut en bas est plus petit qu'un intervalle dans la direction allant de haut en bas entre les éléments élastiques au niveau de la taille (36) adjacents dans la direction allant de haut en bas.

11. Article absorbant en forme de culotte selon l'une quelconque des revendications 1 à 10, dans lequel
une pluralité des éléments élastiques inclinables (37) sont disposés dans la partie d'extension (32) au niveau d'un espace dans la direction allant de gauche à droite,
une valeur maximale d'un espace dans la direction allant de gauche à droite entre une paire se trouvant le plus à l'extérieur dans la direction allant de gauche à droite des éléments élastiques inclinables (37) adjacents l'un par rapport à l'autre dans la direction allant de gauche à droite
est inférieure à
une valeur minimale d'un espace dans la direction allant de gauche à droite entre une paire des éléments élastiques inclinables (37) adjacents l'un par rapport à l'autre dans la direction allant de gauche à droite se trouvant à l'intérieur de la paire se trouvant le plus à l'extérieur dans la direction allant de gauche à droite.

12. Article absorbant en forme de culotte selon l'une quelconque des revendications 1 à 11, dans lequel
une pluralité des éléments élastiques latéraux (38) sont disposés dans la partie d'extension (32) selon un intervalle dans la direction allant de haut en bas, et
une longueur sur laquelle chacun de la pluralité d'éléments élastiques latéraux (38) s'étend vers l'extérieur dans la direction allant de gauche à droite au-delà de l'élément élastique inclinable (37) diffère.

13. Article absorbant en forme de culotte selon l'une quelconque des revendications 1 à 12, dans lequel
dans l'article absorbant en forme de culotte dans un état naturel,
la partie d'extension (32) comprend une partie faisant saillie à l'extérieur de l'élément élastique inclinable (37) dans la direction allant de gauche à droite.
